# EUROPÄISCHE PATENTSCHRIFT

(11) **EP 3 437 673 B1**
(45) Veröffentlichungstag und Bekanntmachung des Hinweises auf die Patenterteilung: **12.02.2020**
(21) Anmeldenummer: 18186864.7
(22) Anmeldetag: 01.08.2018
(51) Int. Cl.: A61M 1/36, A61M 5/36, A61B 5/145, G01N 33/48, G01N 33/487, G01N 33/49, G01N 21/85

(54) **VERFAHREN ZUR DETEKTION VON EINSCHLÜSSEN VON GASEN IN BLUT**
METHOD FOR DETECTING INCLUSIONS OF GASES IN BLOOD
PROCÉDÉ DE DÉTECTION D'INCLUSIONS GAZEUSES DANS LE SANG

(30) Priorität: 01.08.2017 DE 102017117448
(43) Veröffentlichungstag der Anmeldung: 06.02.2019
(73) Patentinhaber: Schwingel, Jörg Hartmut, 66564 Ottweiler (DE)
(72) Erfinder: Schwingel, Jörg Hartmut, 66564 Ottweiler (DE)
(74) Vertreter: Patentanwaltskanzlei Vièl & Wieske PartGmbB

(56) Entgegenhaltungen:
- DE-A1- 1 598 606
- DE-A1- 2 017 112
- US-A1- 2013 134 077

## Beschreibung

Die vorliegende Erfindung betrifft ein Verfahren zur Detektion von Einschlüssen von Gasen im Blut bei einer Dialysebehandlung nach dem Oberbegriff des Anspruchs 1.

Es bekannt, Flüssigkeit durch eine Leitung zu fördern. Dies gilt grundsätzlich für beliebige Flüssigkeiten. Bei dem Verfahren der vorliegenden Erfindung geht es um die Förderung von Blut in einer Leitung im Rahmen der Durchführung einer Dialyse-Behandlung.

Die US 2013 134 077 A1 beschreibt ein Verfahren zur Messung von Gaseinschlüssen im Blut (extrakorporal). Hierzu wird mit einem elektromagnetischen Anregungssignal, das über einen weiten Frequenzbereich durchgewobbelt wird, ein Anregungssignal erzeugt. Es wird eine elektromagnetische Größe gemessen, mit der festgestellt wird, ob sich Gaseinschlüsse in dem Blut befinden. Bei der elektromagnetischen Größe kann es sich auch um ein Magnetfeld handeln.

DE DE-OS 2 017 112 betrifft ein Verfahren, bei dem mit einer kapazitiven Messanordnung Lufteinschlüsse im Blut erkannt werden können, das in einer Leitung strömt. Die Lufteinschlüsse in dem Blut führen zu einer Änderung des Dielektrikums, so dass mittels der kapazitiven Messanordnung die Lufteinschlüsse im Blut erkannt werden können.

Im Rahmen einer Dialyse-Behandlung ist es wichtig zu detektieren, ob Gaseinschlüsse vorhanden sind. Wenn Gaseinschlüsse vorhanden sind, ist es gerade bei einer Dialysebehandlung vorteilhaft zu wissen, um welche Gase es sich handelt und / oder wie hoch der relative Gasanteil in der Flüssigkeit ist. Es lässt sich ein Grenzwert definieren, bis zu dem ein Gasanteil im Blut für den Patienten unkritisch ist. Bei einem Überschreiten des Grenzwertes kann der Gasanteil im Blut für den Patienten kritisch werden, weil das Herz des Patienten nur Blut fördern kann, wenn der Gasanteil des Blutes begrenzt ist.

Der vorliegenden Erfindung liegt die Aufgabe zu Grunde, ein Verfahren vorzuschlagen, mit dem Einschlüsse von Gasen im Blut bei einer Dialysebehandlung detektiert werden können.

Diese Aufgabe wird gemäß Anspruch 1 gelöst, indem ein Magnetfeld unter definierten Bedingungen erzeugt wird, wobei der Leitung ein Sensor zugeordnet ist, mit dem eine die Stärke des Magnetfeldes repräsentierende Größe messbar ist. Die Erzeugung des Magnetfeldes unter definierten Bedingungen erfolgt, indem der Sensor aus zwei miteinander korrespondierenden Spulen besteht, bei dem die erste Spule mit einem Gleichstrom beaufschlagt wird. Die die Stärke des Magnetfeldes repräsentierende Größe wird gemessen, indem das in der zweiten Spule induzierte Magnetfeld ausgewertet wird.

Es hat sich gezeigt, dass Flüssigkeiten abhängig von ihrer Zusammensetzung einen Einfluss auf ein Magnetfeld haben, wenn die Flüssigkeiten strömen. Dazu muss die Flüssigkeit nicht unmittelbar geladene Teilchen enthalten. Das Auftreten eines derartigen Effekts lässt sich auch damit erklären, dass die Moleküle der Flüssigkeit unter Umständen eine unsymmetrische Verteilung der elektrischen Ladung (und damit eine Polarität) aufweisen. Eine weitere Erklärung kann darin bestehen, dass in der Flüssigkeit Bestandteile enthalten sind, die unmittelbar einen Einfluss auf das Magnetfeld haben. Dies gilt beispielsweise für Eisen als Bestandteil der Flüssigkeit.

Ein mit derselben Geschwindigkeit wie die Flüssigkeit strömendes Gas hat durchweg einen anderen Einfluss auf das Magnetfeld.

Daraus resultiert, dass bei einem Gaseinschluss in der Flüssigkeit ein entsprechendes Volumen der Flüssigkeit durch den Gaseinschluss "verdrängt" wird. Das bedeutet wiederum, dass sich der Einfluss der strömenden Flüssigkeit mit dem Gaseinschluss auf das Magnetfeld zusammensetzt aus dem Einfluss der Flüssigkeit auf das Magnetfeld sowie dem Einfluss des strömenden Gases auf das Magnetfeld, wobei die Einflüsse gewichtet werden abhängig von den relativen Volumenanteilen der Flüssigkeit sowie des Gases in der Flüssigkeit mit dem Gaseinschluss. Dies gilt zumindest so lange, wie keine Wechselwirkungen des Einflusses der Flüssigkeit sowie des Gases auf das Magnetfeld auftreten.

Da das fließende Blut zu einer Änderung des Magnetfeldes führt, wird in der zweiten Spule ein sich änderndes Magnetfeld induziert. Dieses sich ändernde Magnetfeld induziert in der zweiten Spule eine Spannung, die gemessen werden kann. Über diese induzierte Spannung kann die Änderung des Magnetfeldes bewertet werden.

In Kenntnis der Erfindung wird noch auf die DE 1 598 606 verwiesen. Es geht dort um strömende Flüssigmetalle. Bei diesen strömenden Flüssigmetallen sollen Einschlüsse von Blasen erkannt werden, indem durch einen Magneten Wirbelströme induziert werden. Diese Wirbelströme werden "gestört", wenn Einschlüsse in der Flüssigkeit (d.h.: dem Flüssigmetall) vorhanden sind.

Bei der Ausgestaltung nach Anspruch 2 besteht der Sensor aus zwei miteinander korrespondierenden Spulen, bei dem die erste Spule geregelt mit einem Gleichstrom beaufschlagt wird derart, dass das in der zweiten Spule induzierte Magnetfeld den Wert "Null" annimmt. Die die Stärke des Magnetfeldes repräsentierende Größe wird aus der Stärke des Gleichstromes abgeleitet, mit dem die erste Spule beaufschlagt wird.

Bei dieser Ausgestaltung wird die Stellgröße, über die das Magnetfeld erzeugt wird, das ein konstantes Magnetfeld in der zweiten Spule induziert, ausgewertet. Das konstante Magnetfeld in der zweiten Spule bedingt, dass das durch die erste Spule erzeugte Magnetfeld unter Beachtung des Einflusses des strömenden Blutes konstant ist. Da durch das strömende Blut ein sich ändernder Einfluss des Magnetfeldes vorliegt, muss sich auch der Strom ändern.

Es hat sich als vorteilhaft erwiesen, zur Erzeugung des Magnetfeldes Gleichstrom zu verwenden. Bei durchgeführten Versuchen zeigte sich hierbei, dass die Messgrößen charakteristische Änderungen abhängig von den Gaseinschlüssen in der Flüssigkeit zeigten, so dass diese gut ausgewertet werden können.

Bei der Ausgestaltung des Verfahrens nach Anspruch 3 werden Referenzmessungen vorgenommen, bei denen die die Stärke des Magnetfeldes repräsentierende Größe jeweils für Blut bestimmt wird, in dem bei den Referenzmessungen definierte Mengen von Gaseinschlüssen vorhanden sind.

Bei den Referenzmessungen wird dem Blut gezielt ein bestimmter Mengenanteil eines bestimmten Gases zugesetzt. Mit diesem Blut mit dem gezielt zugesetzten Gasanteil wird anschließend die Referenzmessung durchgeführt.

Aus einem Vergleich der Messung des zu untersuchenden Blutes mit den Referenzmessungen lässt sich eine Aussage über die Menge des Gases in dem Blut und / oder über die Art des Gases in dem Blut treffen.

Es hat sich gezeigt, dass unter den Bedingungen der vorhergehenden Ansprüche mit der Erzeugung des Magnetfeldes mittels Gleichstroms die induzierte Spannung in der zweiten Spule linear ansteigt (bzw. fällt - abhängig von der Polung des Gleichstromes), mit zunehmendem Gasanteil. Dies gilt für alle untersuchten Gase. Als Flüssigkeit wurde mehrfach entlüftetes Wasser verwendet.

Für die unterschiedlichen Gase zeigten sich dabei unterschiedliche Steigungen der Geraden bezüglich der induzierten Spannung in Abhängig von dem relativen Gasanteil in der Flüssigkeit.

Wenn bekannt ist, welches Gas in dem Blut eingeschlossen ist, lässt sich mit der Messmethode über den Vergleich mit den Referenzmessungen der relative Anteil dieses Gases in dem Blut bestimmen.

Wenn bekannt ist, welcher relative Anteil von Gas in dem Blut enthalten ist, lässt sich mit dieser Messmethode aus einem Vergleich mit den Referenzmessungen bestimmen, um welches Gas es sich handelt. Dies gilt dann, wenn es sich um lediglich eine Gassorte handelt.

Handelt es sich um ein Gemisch von zwei oder mehr Gasen, lässt sich mit dieser Messmethode allein lediglich ein Wertebereich bestimmen, in dem die Einzelbestandteile der Gase relativ zueinander in dem Blut eingeschlossen sein können.

Bei einem Vergleich mit den Referenzmessungen bezüglich der einzelnen untersuchten Gase kann anhand des Anfangs- und / oder Endpunktes des betreffenden Spannungsbereichs eventuell bereits geschlossen werden, dass ein bestimmtes Gas nicht eingeschlossen sein kann. Dies gilt dann, wenn es sich bei dem eingeschlossenen Gas nicht um ein Gasgemisch handelt sondern um ein einziges Gas und die gemessene Spannung außerhalb des Spannungsintervalls liegt, das bei den Referenzmessungen für verschiedenen prozentuale Anteile des betreffenden Gases gemessen wurde.

Dies lässt sich anhand der Betrachtung von zwei verschiedenen Gasen in der Flüssigkeit erläutern. Die beiden Gase haben unterschiedliche lineare Abhängigkeiten hinsichtlich der Steigung der induzierten Spannung über dem jeweiligen relativen Anteil des Gases. Ein bestimmter relativer Anteil des ersten Gases führt zu derselben induzierten Spannung wie ein anderer definierter Anteil des zweiten Gases. Ebenso sind auch "Zwischenwerte" möglich, bei denen ein Gemisch aus dem ersten Gas und dem zweiten Gas vorhanden ist. Ein Gas, das bei einem bestimmten relativen Anteil in dem Blut zu einer bestimmten induzierten Spannung führt, kann "ersetzt" werden durch einen anderen definierten relativen Anteil eines anderen Gases in dem Blut, wobei das andere Gas zu derselben induzierten Spannung führt. Dabei ist es nicht zwingend notwendig, dass das erste Gas vollständig durch das zweite Gas ersetzt wird. Das erste Gas kann auch lediglich teilweise ersetzt werden, so dass ein Gemisch aus dem ersten und dem zweiten Gas vorliegt. Entsprechendes gilt auch, wenn mehr als zwei Gase in dem Gasgemisch vorhanden sind.

Dabei ist Voraussetzung, dass der Einfluss der verschiedenen Gase auf die induzierte Spannung jeweils unabhängig voneinander ist, so dass keine Wechselwirkungen auftreten.

Bei der Ausgestaltung des Verfahrens nach Anspruch 4 befindet sich die Fördergeschwindigkeit des Blutes bei den Referenzmessungen und bei der durchzuführenden Messung in einem definierten Geschwindigkeitsbereich.

Es hat sich gezeigt, dass die Fördergeschwindigkeit einen Einfluss auf das Messergebnis hat. Deswegen ist es sinnvoll, dass dieser Parameter bei den Referenzmessungen im Verhältnis zur Messung an dem zu untersuchenden Blut übereinstimmt.

Bei der Ausgestaltung des Verfahrens nach Anspruch 5 ist die Leitung im Messbereich des Sensors horizontal angeordnet.

Dies erweist sich als vorteilhaft insofern, als die Geschwindigkeit des Blutes einen Einfluss auf das Messergebnis der Auswertung des induktiven Sensors hat. Wenn die Leitung horizontal ist, erfahren die Gaseinschlüsse in dem Blut keinen Auftrieb. Es hat sich gezeigt, dass die Gaseinschlüsse dann gleichförmig mit der Geschwindigkeit des Blutes mit gefördert werden.

Bei einer vertikal angeordneten Leitung (bzw. bei einer Anordnung, bei der die Leitung eine vertikale Komponente hat, wenn diese beispielsweise schräg angeordnet ist), erfahren die Gaseinschlüsse in Folge der Auftriebskräfte gegenüber dem Blut eine Beschleunigung. Die Gaseinschlüsse bewegen sich in einer solchen Anordnung mit einer größeren Geschwindigkeit als das Blut. Hinzu kommt noch, dass die Gaseinschlüsse gegenüber dem Blut beschleunigt bewegt werden.

Aus diesen Gründen erweist es sich als vorteilhaft, die Leitung horizontal anzuordnen im Messbereich, so dass die Gaseinschlüsse möglichst homogen mit dem Blut mitbewegt werden.

Anspruch 6 betrifft eine Ausgestaltung des Verfahrens, bei der zusätzlich ider Leitung in einem Messbereich ein kapazitiver Sensor zugeordnet ist derart, dass sich das Blut im Bereich des elektrischen Feldes des kapazitiven Sensors befindet.

Es hat sich gezeigt, dass das Blut als Dielektrikum des kapazitiven Sensors betrachtet werden kann, wobei die Dielektrizität des Blutes von eventuellen Gaseinschlüssen abhängt.

Bei durchgeführten Versuchen zeigte sich wiederum ein linearer Zusammenhang der Kapazität mit zunehmendem Anteil des Gases in der Flüssigkeit.

Dabei zeigte sich auch, dass sich für denselben relativen Anteil eines Gases für unterschiedliche Gassorten unterschiedliche Kapazitäten ergeben.

Auch bei der Ausgestaltung des Verfahrens nach Anspruch 6 können Referenzmessungen vorgenommen werden, bei denen die sich ergebende Kapazität jeweils für Blut bestimmt wird, in dem bei den Referenzmessungen definierte Mengen von Gaseinschlüssen in der Flüssigkeit vorhanden sind.

Bei den Referenzmessungen wird dem Blut gezielt ein bestimmter Mengenanteil eines bestimmten Gases zugesetzt. Mit diesem Blut mit dem gezielt zugesetzten Gasanteil wird anschließend die Referenzmessung durchgeführt.

Aus einem Vergleich der Messung des zu untersuchenden Blutes mit den Referenzmessungen lässt sich eine Aussage über die Menge des Gases in dem Blut und/oder über die Art des Gases in dem Blut treffen.

Wenn bekannt ist, welches Gas in dem Blut eingeschlossen ist, lässt sich mit der kapazitiven Messmethode über den Vergleich mit den Referenzmessungen eine Aussage über den relativen Anteil dieses Gases in dem Blut treffen.

Wenn bekannt ist, welcher relative Anteil von Gas in dem Blut enthalten ist, lässt sich mit dieser Messmethode aus einem Vergleich mit den Referenzmessungen eine Aussage treffen, um welches Gas es sich handelt. Dies gilt dann, wenn es sich um lediglich eine Gassorte handelt.

Bei einem Vergleich mit den Referenzmessungen bezüglich der einzelnen untersuchten Gase kann anhand der gemessenen Kapazitäten für die einzelnen Gassorten eventuell bereits geschlossen werden, dass ein bestimmtes Gas nicht eingeschlossen sein kann. Dies gilt dann, wenn es sich bei dem eingeschlossenen Gas nicht um ein Gasgemisch handelt sondern um ein einziges Gas und die gemessene Kapazität außerhalb des Wertebereichs der Kapazitäten liegt, innerhalb dem bei den Referenzmessungen für verschiedenen prozentuale Anteile des betreffenden Gases Kapazitäten gemessen wurden.

Handelt es sich um ein Gemisch von zwei oder mehr Gasen, lässt sich mit dieser Messmethode allein lediglich ein Wertebereich bestimmen, in dem die Einzelbestandteile der Gase relativ zueinander in dem Blut eingeschlossen sein können.

Dies lässt sich anhand der Betrachtung von zwei verschiedenen Gasen in dem Blut erläutern. Die beiden Gase haben unterschiedliche lineare Abhängigkeiten hinsichtlich der Änderung der Kapazität über dem jeweiligen relativen Anteil des Gases. Ein bestimmter relativer Anteil des ersten Gases führt zu derselben Änderung der Kapazität wie ein anderer definierter Anteil des zweiten Gases. Ebenso sind auch "Zwischenwerte" möglich, bei denen ein Gemisch aus dem ersten Gas und dem zweiten Gas vorhanden ist. Ein Gas, das bei einem bestimmten relativen Anteil in dem Blut zu einer bestimmten Änderung der Kapazität führt, kann "ersetzt" werden durch einen anderen definierten relativen Anteil eines anderen Gases in dem Blut, wobei das andere Gas zu derselben Änderung der Kapazität führt. Dabei ist es nicht zwingend notwendig, dass das erste Gas vollständig durch das zweite Gas ersetzt wird. Das erste Gas kann auch lediglich teilweise ersetzt werden, so dass ein Gemisch aus dem ersten und dem zweiten Gas vorliegt. Entsprechendes gilt auch, wenn mehr als zwei Gase in dem Gasgemisch vorhanden sind.

Dabei ist Voraussetzung, dass der Einfluss der verschiedenen Gase auf die Änderung der Kapazität jeweils unabhängig voneinander ist, so dass keine Wechselwirkungen auftreten.

Bei der Ausgestaltung des Verfahrens nach Anspruch 7 wird die Kapazität des kapazitiven Sensors aus dem zeitlichen Verhalten der Änderung des Stromes und/ oder der Spannung bei der Aufladung und / oder der Entladung der Kapazität bestimmt.

Hierbei handelt es sich um ein vergleichsweise gut umsetzbares Messverfahren, bei dem die Zeitdauer zwischen sich ergebenden Schwellwerten des Stromes und / oder der Spannung ausgewertet werden können.

Bei der Ausgestaltung nach Anspruch 8 wird das Blut in Kombination mit der Auswertung des Magnetfeldes sowie ggf. weiterhin in Kombination mit der kapazitiven Auswertung durch eine Leitung gefördert, die zumindest in einem Messbereich lichtdurchlässig ist. Gemäß Anspruch 8 wird die Absorption und / oder Streuung des Lichtes ausgewertet, das auf die Leitung gerichtet ist.

Es hat sich gezeigt, dass das Blut abhängig von Einschlüssen von Gasen ein unterschiedliches Verhalten zeigt hinsichtlich der Absorption und / oder Streuung von Licht. Die Absorption und / oder Streuung hängt dabei von der Gassorte ab, von deren relativer Konzentration in dem Blut sowie auch von der Wellenlänge des Lichtes. Durch die Projektion von Licht, das Bestandteile mit mehreren Wellenlängen aufweist, lassen sich somit mehrere voneinander unabhängige Messungen durchführen, so dass damit mehrere der Parameter bestimmt werden können (Bestimmung unterschiedlicher Gassorten sowie Bestimmung der relativen Anteile der Gassorten in der Flüssigkeit).

Gemäß Anspruch 9 wird das zu untersuchende Blut mit Licht zumindest in einem definierten Wellenlängenbereich beaufschlagt derart, dass das Licht aufgeteilt wird in einen ersten Teil, der - ohne das zu untersuchende Blut zu durchdringen - auf einen Referenzsensor geleitet wird und in einen zweiten Teil, der das Blut durchdringt und auf einen Messsensor geleitet wird.

Gerade bei der Verwendung von Licht mit unterschiedlichen Wellenlängen erweist es sich als vorteilhaft, dass die verschiedenen Gase für das Licht abhängig von der jeweiligen Wellenlänge eine unterschiedliche Absorption bzw. Streuung aufweisen. Damit lassen sich aus einer wellenlängenabhängigen Auswertung Aussagen darüber treffen, welche Gassorten ggf. in dem Blut vorhanden sind.

Auch bei der Ausgestaltung des Verfahrens nach Anspruch 9 können Referenzmessungen vorgenommen werden, bei denen die sich ergebenden Differenzen der Signale des Referenzsensors zu den Signalen des Messsensors jeweils für Blut bestimmt werden, in dem bei den Referenzmessungen definierte Mengen von Gaseinschlüssen vorhanden sind.

Bei den Referenzmessungen wird dem Blut gezielt ein bestimmter Mengenanteil eines bestimmten Gases zugesetzt. Mit diesem Blut mit dem gezielt zugesetzten Gasanteil wird anschließend die Referenzmessung durchgeführt.

Aus einem Vergleich der Messung der Differenz(en) der Signale des Messsensors zu den Signal(en) des Referenzsensors für das zu untersuchende Blut mit den entsprechenden Differenzen der Signale des Messsensors zu den Signalen des Referenzsensors für die Referenzmessungen lässt sich eine Aussage über die Menge des Gases in dem Blut und/oder über die Art des Gases in dem Blut treffen.

Durch die Auswertung der Absorption und/oder Streuung des Lichtes bei unterschiedlichen Wellenlängen lässt sich eine Aussage über den relativen Anteil dieses Gases in dem Blut treffen und/oder eine Aussage darüber, welches Gas in dem Blut enthalten ist.

Handelt es sich um ein Gemisch von zwei oder mehr Gasen, lässt sich mit dieser Messmethode zumindest ein Wertebereich bestimmen, in dem die Einzelbestandteile der Gase relativ zueinander in dem Blut eingeschlossen sein können. Abhängig von den ermittelten Ergebnissen bei einer Referenzmessung können sich auch für unterschiedliche Wellenlängenbereiche des Lichtes Messwerte derart ergeben, dass auch die Einzelbestandteile von Gasen in einem Gasgemisch eindeutig erkannt werden können. Bei den durchgeführten Versuchen mit destilliertem und mehrfach entlüftetem Wasser zeigten sich für verschiedene Gassorten (CO₂, O₂, medizinischer Sauerstoff) deutliche Unterschiede bei Wellenlängen in den Bereichen um 400 nm, um 500 nm, um 680 nm, so dass die Gassorten als solche sowie auch hinsichtlich der vorhandenen Menge detektiert werden konnten.

Dies lässt sich anhand der Betrachtung von zwei verschiedenen Gasen in der Flüssigkeit erläutern. Die beiden Gase haben unterschiedlichen Einfluss auf die Absorption und/oder Streuung des Lichtes. Die Absorption und/oder Streuung hängt weiterhin von dem jeweiligen relativen Anteil des Gases ab. Ein bestimmter relativer Anteil des ersten Gases führt zu derselben Änderung der Absorption und/oder Streuung des Lichtes wie ein anderer definierter Anteil des zweiten Gases. Ebenso sind auch "Zwischenwerte" möglich, bei denen ein Gemisch aus dem ersten Gas und dem zweiten Gas vorhanden ist. Ein Gas, das bei einem bestimmten relativen Anteil im Blut zu einer bestimmten Absorption und/oder Streuung des Lichtes in einem bestimmten Wellenlängenbereich des Lichtes führt, kann "ersetzt" werden durch einen anderen definierten relativen Anteil eines anderen Gases im Blut, wobei das andere Gas zu derselben Änderung der Absorption und/oder Streuung des Lichtes in diesem Wellenlängenbereich führt. Bei den anderen Messverfahren (induktives Messverfahren, kapazitives Messverfahren), bei denen die Änderung der gemessenen Größe mit dem jeweiligen relativen Anteil des Gases eine lineare Abhängigkeit zeigte, war die Menge des anderen Gases konstant, die eine bestimmte Menge des ersten Gases ersetzt hat und dabei dieselbe Auswirkung auf die Messgröße hatte. Bei der optischen Methode ist dieses Verhältnis unterschiedlich für die einzelnen Wellenlängenbereiche. Dabei ist es nicht zwingend notwendig, dass das erste Gas vollständig durch das zweite Gas ersetzt wird. Das erste Gas kann auch lediglich teilweise ersetzt werden, so dass ein Gemisch aus dem ersten und dem zweiten Gas vorliegt. Entsprechendes gilt auch, wenn mehr als zwei Gase in dem Gasgemisch vorhanden sind.

Dabei ist Voraussetzung, dass der Einfluss der verschiedenen Gase auf die Änderung der Absorption / Streuung jeweils unabhängig voneinander ist, so dass keine Wechselwirkungen auftreten.

Durch eine Auswertung unterschiedlicher Wellenlängenbereiche lässt sich eingrenzen, um welche Gase mit welchen relativen Anteilen in der Flüssigkeit es sich handelt.

Es hat sich dabei gezeigt, dass sowohl hinsichtlich der Detektion der Größe von Gasblasen als auch hinsichtlich der Auswertung, um welche Art von Gasen es sich handelt, eine Kombination des kapazitiven Messverfahrens mit dem optischen Messverfahren geeignet ist.

Das Blut hat unterschiedliche Zusammensetzungen, die sich auch auf die beschriebenen Messmethoden auswirken können. Diese unterschiedlichen Zusammensetzungen können beispielsweise abhängig von der aktuellen körperlichen oder psychischen Belastung sein oder auch abhängig davon, zu welcher Zeit vor der Untersuchung der Patient eventuell noch eine Mahlzeit zu sich genommen hat und woraus diese Mahlzeit bestand.

Es kann sinnvoll sein, diese unterschiedlichen Situationen zu berücksichtigen, indem zu den verschiedenen Bedingungen jeweils Referenzmessungen vorgenommen werden, so dass dann bei einer durchzuführenden Untersuchung eine Referenzmessung als Vergleich herangezogen werden kann.

Als eine sinnvolle Sensor-Kombination hat sich eine Kombination des kapazitiven Verfahrens zur Klassifizierung der betrachteten Gasmedien im Detektionsvolumen und das optische Verfahren als Detektionsmöglichkeit im Hinblick auf Mikroblasen gezeigt.

Diese Messverfahren können weiterhin kombiniert werden mit der Sonographie. Die Detektion von Gaseinschlüssen mittels akustischer Methoden ist dadurch bedingt, dass Gas den Schall sehr schlecht (gegenüber einer Flüssigkeit) leitet. An den Übergangsstellen Medium-Gas-Medium setzen Reflexionen des Schalls bis hin zu Streuungen ein.

Die bekannten Messverfahren zur Erfassung des Blutdruckes sind durchweg berührungsbehaftete Verfahren. Schonender für den Patienten ist jedoch die Erfassung mittels eines berührungslosen Verfahrens. Dies kann beispielsweise auch ein Verfahren sein, das sich der beschriebenen kapazitiven und / oder induktiven Effekte bedient.

Derzeit erfolgt die Erfassung des pH-Wertes von menschlichem Blut ebenfalls noch nicht berührungslos, sondern mittels einer Messsonde im Rahmen eines chemischen Verfahrens. Auch hierbei besteht die Möglichkeit, den pH-Wert des Blutes über einen weiteren gekoppelten Effekt (nicht nur mittels Temperaturerfassung nach Widerstandsänderung) berührungslos zu erfassen. Dabei ist es sehr hilfreich, dass der CO₂-Spiegel im Blut abhängig ist vom pH-Wert und umgekehrt. Eine entsprechende Beziehung ergibt sich über die O₂-Aufnahmefähigkeit des Blutes (besser Hämoglobin), denn mit steigendem pH-Wert erhöht sich auch die O₂ - Aufnahmefähigkeit des Hämoglobins. Wird der O₂-Gehalt im Blut berührungsfrei festgestellt, so kann damit auch eine Aussage über den Kohlenstoffdioxidanteil und somit auch über den aktuellen pH-Wert getroffen werden. Hierzu kann die Kapnometrie einen wesentlichen Beitrag leisten. Im Rahmen der Messverfahren in der Rheologie kann über die Fließgeschwindigkeit des Blutes sowohl eine Aussage über die aktuelle Zusammensetzung (Eigenschaften der Bestandteile des Blutes) als auch über die Effektivität des O₂-Transportes im Hinblick auf die Mikrozirkulation (Versorgung mit O₂ bis in die kleinsten Kapillaren), getroffen werden.

Aber nicht nur in der Medizintechnik können die erhaltenen Forschungsergebnisse Anwendung finden. In den durchgeführten Versuchen mithilfe der betrachteten Verfahren können die einzelnen Medien beliebig ausgetauscht und für viele weitere Anwendungsfalle, bei denen Gaseinschlüsse unerwünscht sind, messtechnisch genutzt werden.

Ein Ausführungsbeispiel der Erfindung ist in der Zeichnung dargestellt. Es zeigt dabei:
- Fig. 1:: Darstellung der induzierten Spannung über den relativen Gasanteil für Umgebungsluft,
- Fig. 2:: Darstellung der induzierten Spannung über den relativen Gasanteil für Sauerstoff,
- Fig. 3:: Darstellung der induzierten Spannung über den relativen Gasanteil für CO₂,
- Fig. 4:: Eine Prinzipdarstellung der Anordnung für eine kapazitive Messung,
- Fig. 5:: Darstellung der Kapazität über dem relativen Gasanteil für O₂,
- Fig. 6:: Darstellung der Kapazität über dem relativen Gasanteil für CO₂,
- Fig. 7:: eine Messanordnung für ein optisches Verfahren ,
- Fig. 8:: Messsignale für ein optisches Verfahren für Wasser und für Umgebungsluft,
- Fig. 9:: Messsignale für ein optisches Verfahren für Wasser und für medizinischen Sauerstoff,
- Fig. 10:: Messsignale für ein optisches Verfahren für Wasser und für CO₂.

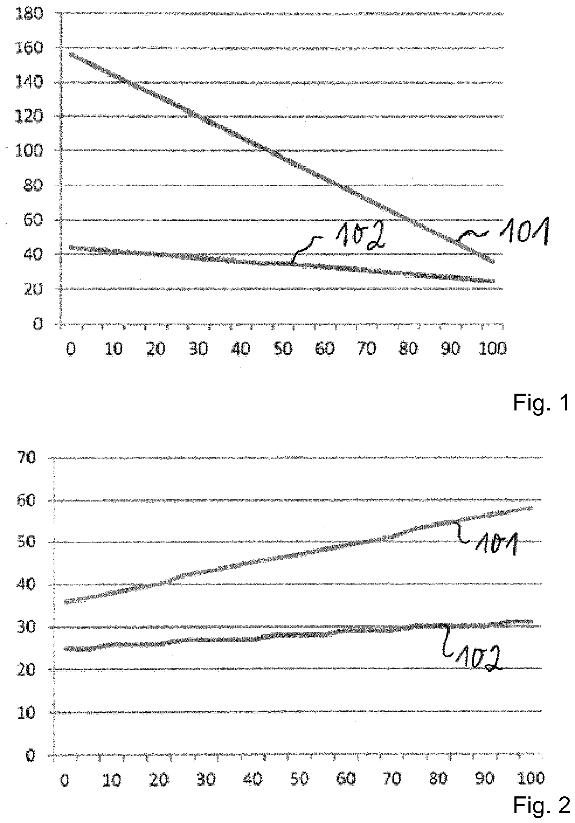

Figur 1 zeigt die induzierte Spannung bei ineinander geschachtelten Spulen, die die Leitung umschließen, in der die Flüssigkeit fließt. Es sind zwei Geraden dargestellt, die für zwei unterschiedliche Spulensysteme 101 und 102 aufgenommen wurden.

Aufgetragen ist die induzierte Spannung in mV über dem relativen Anteil von Umgebungsluft in der Flüssigkeit (destilliertes Wasser, mehrfach entlüftet) in Prozent. Der Gasanteil wurde der Flüssigkeit jeweils gezielt zugegeben.

Figur 2 zeigt die induzierte Spannung bei ineinander geschachteten Spulen, die die Leitung umschließen, für O₂.

Aufgetragen ist die induzierte Spannung in mV über dem relativen Anteil von Sauerstoff in der Flüssigkeit (destilliertes Wasser, mehrfach entlüftet) in Prozent. Der Sauerstoffanteil wurde der Flüssigkeit jeweils gezielt zugegeben.

Es sind wieder die Geraden für die beiden Spulensysteme 101 und 102 dargestellt.

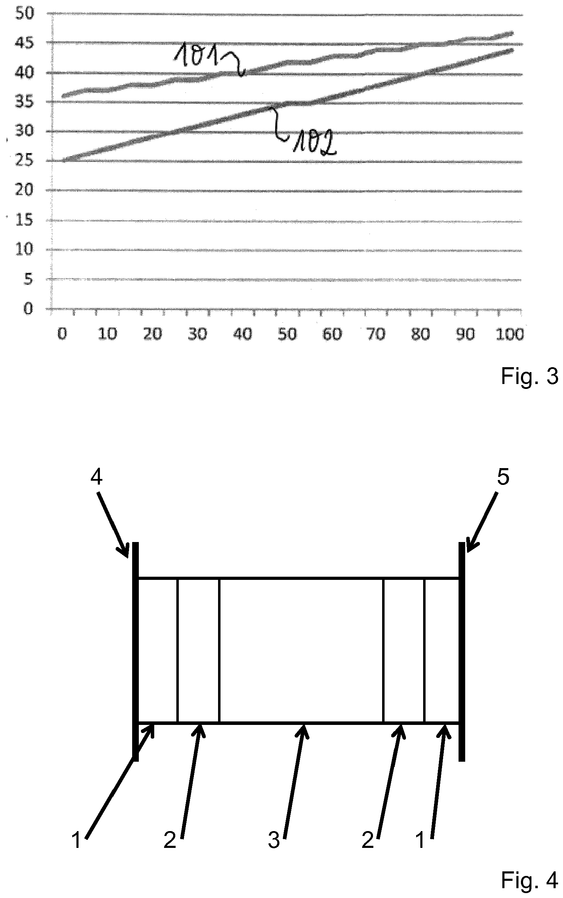

Figur 3 zeigt die induzierte Spannung bei ineinander geschachteten Spulen, die die Leitung umschließen, für CO₂.

Aufgetragen ist die induzierte Spannung in mV über dem relativen Anteil von Kohlendioxid in der Flüssigkeit (destilliertes Wasser, mehrfach entlüftet) in Prozent. Der Anteil des CO₂ wurde der Flüssigkeit jeweils gezielt zugegeben.

Es sind wieder die Geraden für die beiden Spulensysteme 101 und 102 dargestellt.

Bei den Figuren 1 bis 3 wurde die Messung jeweils mit Gleichstrom durchgeführt.

Die fallende Gerade der Figur 1 im Verhältnis zu den steigenden Geraden bei den Figuren 2 und 3 erklärt sich aus den unterschiedlichen Polungsrichtungen des Gleichstroms.

Die Figur 4 zeigt eine Prinzipdarstellung des Kondensators. Die Bezugsziffer 3 zeigt die innere Öffnung der Leitung, in der die Flüssigkeit fließt (in der Zeichnungsebene senkrecht nach oben bzw. unten. Die Leitung wird durch ein Rohr gebildet, dessen Wand mit der Bezugsziffer 2 gekennzeichnet ist. Diese Wand kann aus Glas bestehen oder auch aus einem PE-Schlauch.

Zur elektrischen Isolierung der beiden Kondensatorplatten 4 und 5 ist auf die Leitung noch eine Teflonschicht 1 aufgebracht.

Figur 4 zeigt einen Querschnitt durch die Leitung in Längsrichtung. Die Leitung ist zylinderförmig und die Teflonschicht sowie die Kondensatorplatten umgeben die Leitung.

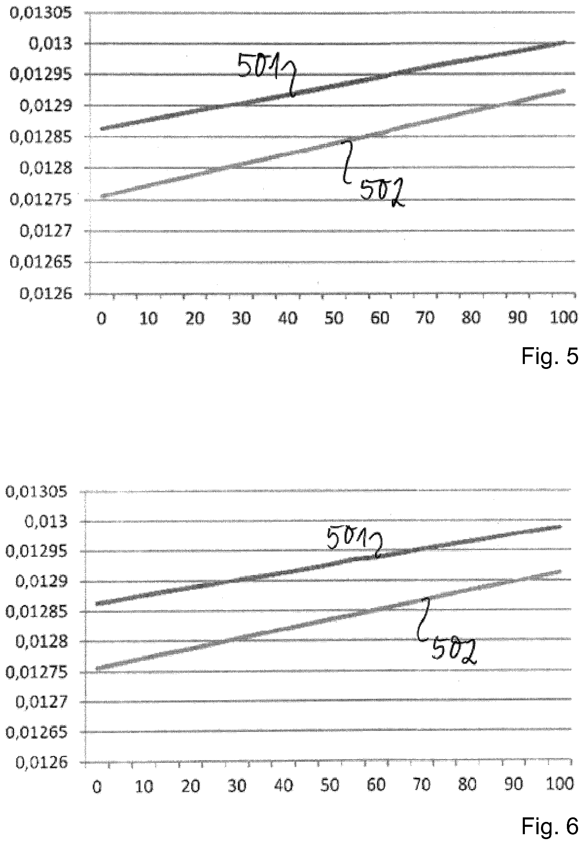

Die Figur 5 zeigt die gemessene Kapazität in nF, aufgetragen über dem relativen Anteil von O₂ in der Flüssigkeit.

Es wurden wieder zwei Kapazitäten 501 und 502 vermessen, zu denen jeweils die sich ergebende Gerade in dem Diagramm eingetragen ist.

Die Figur 6 zeigt die gemessene Kapazität in nF, aufgetragen über dem relativen Anteil von CO₂ in der Flüssigkeit.

Es wurden wieder zwei Kapazitäten 501 und 502 vermessen, zu denen jeweils die sich ergebende Gerade in dem Diagramm eingetragen ist.

Ergänzend und vergleichbar zu den Diagrammen der Figuren 5 und 6 können entsprechende Diagramme für weitere Gase erstellt werden.

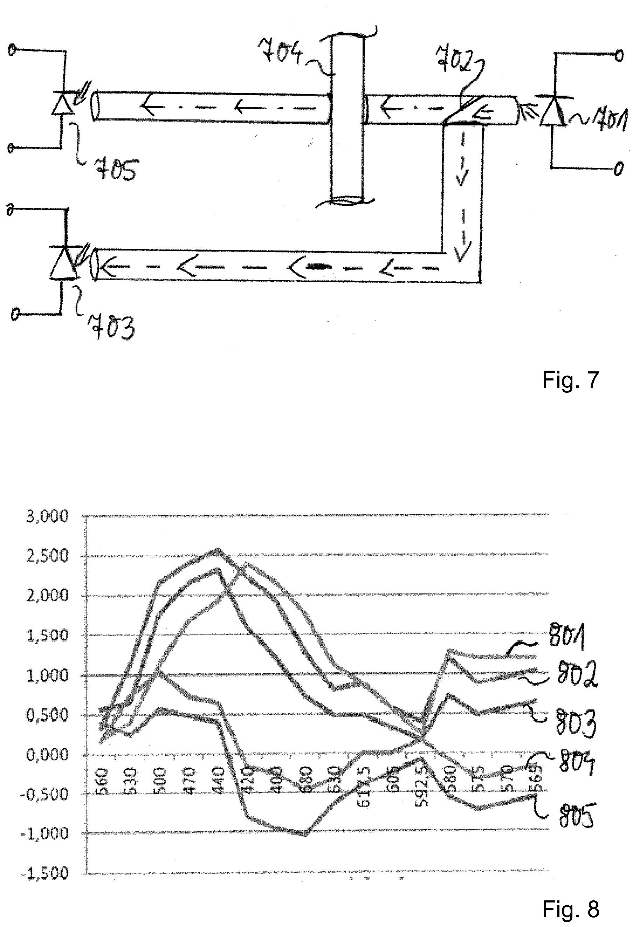

Die Figur 7 zeigt eine Anordnung für ein optisches Messverfahren. Von der Leuchtdiode 701 wird Licht ausgesendet. Dieses Licht kann weitgehend monochromatisch sein oder auch Bestandteile unterschiedlicher Wellenlängenbereich aufweisen (ggf. auch Licht über den gesamten Bereich des sichtbaren Spektrums).

Das Licht von der Leuchtdiode 701 trifft auf einen Strahlteiler 702. Dort wird das Licht in einen Bestandteil aufgeteilt, der ohne durch die zu untersuchende Flüssigkeit geleitet zu werden direkt auf einen Referenzsensor 703 geleitet wird. Der andere Bestandteil des Lichtes wird durch die zu untersuchende Flüssigkeit geleitet. Dazu kann das Licht eine entsprechende Leitung 704, in der die Flüssigkeit strömt, quer zur Längsrichtung der Leitung durchdringen. Dieses Licht trifft dann auf einen Messsensor 705 auf. Durch einen Vergleich der Signale des Referenzsensors 703 zu dem Messsensor 705 lässt sich die Absorption und / oder Streuung des Lichtes erfassen.

Die Figur 8 zeigt Spannungsverläufe aufgetragen über verschiedenen Wellenlängen des Lichtes zum einen für Wasser (destilliert, mehrfach entlüftet) sowie für Umgebungsluft.

Die Kurven wurden mit einer Messanordnung nach Figur 7 aufgenommen. Die Kurve 801 entspricht der Referenzdetektorspannung an dem Referenzsensor 703. Die Kurve 802 entspricht dem Spannungsverlauf an dem Messsensor 705, wenn die Leitung 704 mit Wasser (destilliert, mehrfach entlüftet) durchströmt wird. Die Kurve 803 entspricht dem Spannungsverlauf an dem Messsensor 705, wenn die Leitung 704 mit Umgebungsluft durchströmt wird.

Die Kurve 804 entspricht der Differenz, die aus dem Spannungsverlauf an dem Messsensor 705 zum Spannungsverlauf an dem Referenzsensor 703 gebildet wird, wenn die Leitung 704 mit Wasser durchströmt wird.

Die Kurve 805 entspricht der Differenz, die aus dem Spannungsverlauf an dem Messsensor 705 zum Spannungsverlauf an dem Referenzsensor 703 gebildet wird, wenn die Leitung 704 mit Umgebungsluft durchströmt wird.

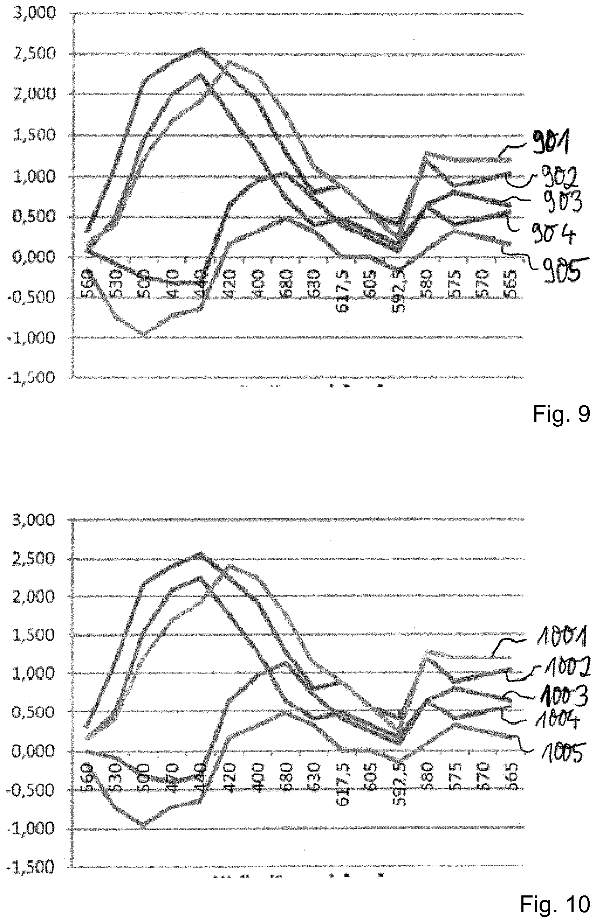

Die Figur 9 zeigt die Darstellung von Spannungsverläufen von Wasser sowie von medizinischem Sauerstoff, aufgetragen über der Wellenlänge des Lichtes.

Die Kurven wurden mit einer Messanordnung nach Figur 7 aufgenommen. Die Kurve 901 entspricht der Referenzdetektorspannung an dem Referenzsensor 703. Die Kurve 902 entspricht dem Spannungsverlauf an dem Messsensor 705, wenn die Leitung 704 mit Wasser (destilliert, mehrfach entlüftet) durchströmt wird. Die Kurve 903 entspricht dem Spannungsverlauf an dem Messsensor 705, wenn die Leitung 704 mit Umgebungsluft durchströmt wird.

Die Kurve 904 entspricht der Differenz, die aus dem Spannungsverlauf an dem Messsensor 705 zum Spannungsverlauf an dem Referenzsensor 703 gebildet wird, wenn die Leitung 704 mit Wasser durchströmt wird.

Die Kurve 905 entspricht der Differenz, die aus dem Spannungsverlauf an dem Messsensor 705 zum Spannungsverlauf an dem Referenzsensor 703 gebildet wird, wenn die Leitung 704 mit medizinischem Sauerstoff durchströmt wird.

Die Figur 10 zeigt die Darstellung von Spannungsverläufen von Wasser sowie von CO₂, aufgetragen über der Wellenlänge des Lichtes.

Die Kurven wurden mit einer Messanordnung nach Figur 7 aufgenommen. Die Kurve 1001 entspricht der Referenzdetektorspannung an dem Referenzsensor 703. Die Kurve 1002 entspricht dem Spannungsverlauf an dem Messsensor 705, wenn die Leitung 704 mit Wasser (destilliert, mehrfach entlüftet) durchströmt wird. Die Kurve 1003 entspricht dem Spannungsverlauf an dem Messsensor 705, wenn die Leitung 704 mit Umgebungsluft durchströmt wird.

Die Kurve 1004 entspricht der Differenz, die aus dem Spannungsverlauf an dem Messsensor 705 zum Spannungsverlauf an dem Referenzsensor 703 gebildet wird, wenn die Leitung 704 mit Wasser durchströmt wird.

Die Kurve 1005 entspricht der Differenz, die aus dem Spannungsverlauf an dem Messsensor 705 zum Spannungsverlauf an dem Referenzsensor 703 gebildet wird, wenn die Leitung 704 mit CO₂ durchströmt wird.

Um eine ausreichende Zahl von Referenzmessungen zur Verfügung zu haben, müssen die Figuren 8 bis 10 noch für verschiedene relative Anteile der jeweiligen Gase in der Flüssigkeit parametriert werden. Durch Interpolation lassen sich dann aus den Referenzmessungen Vergleiche zu realen Messungen ziehen.

Bei den Figuren 8 bis 10 ergeben sich entsprechend deutliche Unterschiede in den Differenzspannungen für Umgebungsluft, Sauerstoff und CO₂ bei den Wellenlängen 400 nm, 500 nm und 680 nm.

## Patentansprüche

1. Verfahren zur Detektion von Einschlüssen von Gasen im Blut bei einer Dialysebehandlung, wobei das Blut durch eine Leitung gefördert wird, wobei ein Magnetfeld unter definierten Bedingungen erzeugt wird, wobei der Leitung ein Sensor zugeordnet ist, mit dem eine die Stärke des Magnetfeldes repräsentierende Größe messbar
ist (101, 102), **wobei** der Sensor aus zwei miteinander korrespondierenden Spulen besteht und **gekennzeichnet dadurch, dass** die erste Spule mit einem Gleichstrom beaufschlagt wird, wobei die die Stärke des Magnetfeldes repräsentierende Größe gemessen wird, indem das in der zweiten Spule induzierte Magnetfeld ausgewertet wird (101, 102).

2. Verfahren nach Anspruch 1,
**dadurch gekennzeichnet, dass** der Sensor aus zwei miteinander korrespondierenden Spulen besteht, bei dem die erste Spule geregelt mit einem Gleichstrom beaufschlagt wird derart, dass das in der zweiten Spule induzierte Magnetfeld den Wert "Null" annimmt, wobei die die Stärke des Magnetfeldes repräsentierende Größe aus der Stärke des Gleichstromes abgeleitet wird, mit dem die erste Spule beaufschlagt wird.

3. Verfahren nach Anspruch 1 oder 2,
**dadurch gekennzeichnet, dass** Referenzmessungen vorgenommen werden, bei denen die die Stärke des Magnetfeldes repräsentierende Größe jeweils für Blut bestimmt wird, in dem bei den Referenzmessungen definierte Mengen von Gaseinschlüssen vorhanden sind.

4. Verfahren nach Anspruch 3,
**dadurch gekennzeichnet, dass** sich die Fördergeschwindigkeit des Blutes bei den Referenzmessungen und bei der durchzuführenden Messung in einem definierten Geschwindigkeitsbereich befindet.

5. Verfahren nach einem der Ansprüche 1 bis 4,
**dadurch gekennzeichnet, dass** die Leitung im Messbereich des Sensors horizontal angeordnet ist.

6. Verfahren nach einem der Ansprüche 1 bis 5,
**dadurch gekennzeichnet, dass** der Leitung in einem Messbereich ein kapazitiver Sensor (4, 5) zugeordnet ist derart, dass sich das Blut (3) im Bereich des elektrischen Feldes des kapazitiven Sensors (4, 5) befindet.

7. Verfahren nach Anspruch 6,
**dadurch gekennzeichnet, dass** die Kapazität des kapazitiven Sensors aus dem zeitlichen Verhalten der Änderung des Stromes und / oder der Spannung bei der Aufladung und / oder der Entladung der Kapazität bestimmt wird (501, 502).

8. Verfahren nach Anspruch 7,
**dadurch gekennzeichnet, dass** die Leitung (704) zumindest in einem Messbereich lichtdurchlässig ist und dass die Absorption und / oder Streuung des Lichtes ausgewertet wird, das auf die Leitung (704) gerichtet ist.

9. Verfahren nach Anspruch 8,
**dadurch gekennzeichnet, dass** das Blut mit Licht zumindest in einem definierten Wellenlängenbereich beaufschlagt wird (701) derart, dass das Licht aufgeteilt wird (702) in einen ersten Teil, der ohne die zu untersuchende Flüssigkeit zu durchdringen auf einen Referenzsensor (703) geleitet wird und in einen zweiten Teil, der das Blut durchdringt und auf einen Messsensor (705) geleitet wird.

## Claims

1. Method for detecting inclusions of gases in the blood in a dialysis treatment, wherein the blood is conveyed through a line, wherein a magnetic field is generated under defined conditions, wherein a sensor is associated with the line, with which sensor a magnitude representing the strength of the magnetic field is measurable (101, 102), wherein the sensor consists of two coils corresponding with one another and **characterised in that** the first coil is acted upon by a direct current, wherein the magnitude representing the strength of the magnetic field is measured by evaluating the magnetic field induced in the second coil (101, 102).

2. Method according to claim 1,
**characterised in that** the sensor consists of two coils corresponding with one another, in which the first coil is acted upon in a controlled manner by a direct current such that the magnetic field induced in the second coil assumes the value "zero", wherein the magnitude representing the strength of the magnetic field is deduced from the strength of the direct current acting upon the first coil.

3. Method according to claim 1 or 2,
**characterised in that** reference measurements are carried out in which the magnitude representing the strength of the magnetic field is determined respectively for blood in which defined quantities of gas inclusions are present in the reference measurements.

4. Method according to claim 3,
**characterised in that** the conveying speed of the blood in the reference measurements and in the measurement to be carried out is located in a defined speed range.

5. Method according to any one of claims 1 to 4,
**characterised in that** the line is arranged horizontally in the measuring region of the sensor.

6. Method according to any one of claims 1 to 5,
**characterised in that** a capacitive sensor (4, 5) is associated with the line in a measuring region such that the blood (3) is located in the region of the electrical field of the capacitive sensor (4, 5).

7. Method according to claim 6,
**characterised in that** the capacitance of the capacitive sensor is determined from the temporal behaviour of the change of the current and / or the voltage in the charging and / or discharging of the capacitor (501, 502).

8. Method according to claim 7,
**characterised in that** the line (704) is light-permeable at least in one measuring region and that the absorption and / or scatter of the light that is directed onto the line (704) is evaluated.

9. Method according to claim 8,
**characterised in that** the blood is acted upon by light at least in a defined wavelength range (701) such that the light is divided (702) into a first part, which is conducted to a reference sensor (703) without penetrating the liquid to be examined, and into a second part, which penetrates the blood and is conducted to a measuring sensor (705).

## Revendications

1. Procédé de détection d'inclusions de gaz dans le sang lors d'un traitement de dialyse, le sang étant transporté par une conduite, un champ magnétique étant généré dans des conditions définies, un capteur étant associé à la conduite avec lequel une grandeur représentant l'intensité du champ magnétique peut être mesurée (101, 102), le capteur étant constitué de deux bobines correspondant l'une à l'autre et **caractérisé en ce qu'**un courant continu est appliqué à la première bobine, la grandeur représentant l'intensité du champ magnétique étant mesurée par exploitation (101, 102) du champ magnétique induit dans la seconde bobine.

2. Procédé selon la revendication 1, **caractérisé en ce que** le capteur comprend deux bobines correspondant l'une à l'autre, dans lequel un courant continu est appliqué à la première bobine de manière contrôlée de telle sorte que le champ magnétique induit dans la seconde bobine prend la valeur "zéro", la grandeur représentant l'intensité du champ magnétique étant déduite de l'intensité du courant continu appliqué à la première bobine.

3. Procédé selon la revendication 1 ou 2, **caractérisé en ce que** des mesures de référence sont effectuées dans lesquelles la grandeur représentant l'intensité du champ magnétique est déterminée dans chaque cas pour du sang dans lequel des quantités définies d'inclusions de gaz sont présentes dans les mesures de référence.

4. Procédé selon la revendication 3, **caractérisé en ce que** la vitesse de transport du sang se situe dans une plage de vitesse définie pour les mesures de référence et pour la mesure à effectuer.

5. Procédé selon l'une des revendications 1 à 4, **caractérisé en ce que** la conduite est disposée horizontalement dans la zone de mesure du capteur.

6. Procédé selon l'une des revendications 1 à 5, **caractérisé en ce qu'**un capteur capacitif (4, 5) est associé à la conduite dans une zone de mesure de telle sorte que le sang (3) est situé dans la région du champ électrique du capteur capacitif (4, 5).

7. Procédé selon la revendication 6, **caractérisé en ce que** la capacité du capteur capacitif est déterminée (501, 502) à partir du comportement temporel de la variation du courant et/ou de la tension pendant la charge et/ou la décharge de la capacité.

8. Procédé selon la revendication 7, **caractérisé en ce que** la conduite (704) est transparente au moins dans une plage de mesure, et **en ce que** l'absorption et/ou la diffusion de la lumière dirigée vers la conduite (704) sont évaluées.

9. Procédé selon la revendication 8, **caractérisé en ce que** le sang est soumis (701) à de la lumière au moins dans une plage de longueurs d'onde définie de telle sorte que la lumière est divisée (702) en une première partie, qui est dirigée sur un capteur de référence (703) sans traverser le liquide à examiner, et en une seconde partie, qui traverse le sang et est dirigée sur un capteur de mesure (705).
